# EUROPEAN PATENT SPECIFICATION

(11) **EP 3 808 732 B1**
(45) Date of publication and mention of the grant of the patent: **09.10.2024**
(21) Application number: 19822219.2
(22) Date of filing: 03.06.2019
(51) Int. Cl.: C07C 263/10, C07C 263/20, C07C 265/14

(54) **METHOD FOR PREPARING ALIPHATIC ISOCYANATES**
VERFAHREN ZUR HERSTELLUNG VON ALIPHATISCHEN ISOCYANATEN
PROCÉDÉ DE PRÉPARATION D'ISOCYANATES ALIPHATIQUES

(30) Priority: 18.06.2018 KR 20180069825
(43) Date of publication of application: 21.04.2021
(73) Proprietor: Hanwha Solutions Corporation, Jung-gu Seoul 04541 (KR)
(72) Inventor: RYU, Hyun Cheol, Daejeon 34127 (KR); HAN, Kee Do, Daejeon 34094 (KR)
(74) Representative: Berggren Oy
(86) International application number: PCT/KR2019/006667
(87) International publication number: WO 2019/245192

(56) References cited:
- KR-A- 20070 110 204
- KR-A- 20120 038 435
- KR-A- 20160 138 410
- KR-A- 20180 058 746
- KR-B1- 101 854 429
- US-B2- 8 563 768
- US-B2- 9 840 461

## Description

### BACKGROUND OF THE INVENTION

### (a) Field of the Invention

The present invention relates to a method for preparing aliphatic isocyanate. More specifically, the present invention relates to a high purity aliphatic isocyanate preparation method that can effectively recover unreacted materials generated during a phosgenation reaction and recycle them to a reaction step, thereby reducing energy consumed when separating reactants.

### (b) Description of the Related Art

Although xylylene diisocyanate(XDI) includes an aromatic ring, it is classified as aliphatic isocyanate, and it is a compound very useful for raw materials such as polyurethane-based material, polyurea-based material or polyisocyanurate-based material, and the like in the fields of chemical industry, resin industry and paint industry.

Commonly, when synthesizing aliphatic isocyanate, many side reactions are generated, and thus, it is prepared by a method of reacting with anhydrous hydrochloric acid or carbonate to form a salt and reacting it with phosgene. For example, XDI is prepared by reacting xylylene diamine(XDA) with anhydrous hydrochloric acid to form an amine-hydrochloric acid salt and reacting it with phosgene. More specifically, in the prior art, aliphatic isocyanate such as XDI was prepared by reacting liquid raw material amine, for example, an XDA containing solution with anhydrous hydrochloric acid to form a XDA-HCl salt, and heating it to a high temperature of 100°C or more, and then, introducing gas phase phosgene to progress a gas-liquid reaction.

As such, the reaction was progressed under high temperature heating because the reaction of forming aliphatic isocyanate is a representative endothermic reaction, and continuous heating and maintenance of a high temperature are required during the reaction so as to increase the yield.

However, since aliphatic isocyanate such XDI generally has high reactivity of the amino group, many side reactions are generated during the phosgenation reaction, and the impurities formed through the side reactions have an influence on the reaction of forming polyurethane resin, thus causing deterioration of resin quality.

As explained above, due to the need to maintain a high temperature during the preparation process of aliphatic isocyanate, and the high reactivity of the produced aliphatic isocyanate such as XDI, there has been a growing concern about the generation of side reactions or production of side products by the thermal denaturation of products, and thus, high load has been frequently generated in the purification process.

Patent publication US 8563768 B2 discloses a process for the preparation of isocyanates by reacting primary amines with a stoichiometric excess of phosgene in the gas phase, wherein the excess phosgene is then recovered and recycled into the reaction.

Patent publication US 9840461 B2 discloses a process for operating a gas phase phosgenation plant to form an isocyanate by reacting an amine with phosgene, in which the gas phase phosgenation plant is started up by first charging the plant with phosgene. At the same time as, or after the first charge of phosgene, the amine supply devices are rendered inert using a hot inert gas stream. Then, amine is admixed for the first time.

### SUMMARY OF THE INVENTION

It is an object of the present invention to provide a method for preparing high purity aliphatic isocyanate that can effectively separate and recover unreacted materials in the later stage of a reaction and recycle them to a reaction step, thereby reducing energy consumed when separating reactants.

Thus, one aspect of the present invention provides a method for preparing aliphatic isocyanate comprising the steps of:
progressing a phosgenation reaction by reacting an aliphatic amine salt and phosgene in the presence of a solvent;
separating aliphatic isocyanate, which is a product in the reaction mixture of the phosgenation reaction step, and sending a mixture comprising hydrogen chloride, unreacted phosgene and a solvent to a condenser to condense;
sending the condensed mixture to a scrubber to discharge hydrogen chloride to the upper stage and separate unreacted phosgene and solvent to the lower stage;
sending the separated unreacted phosgene and solvent to a distillation column to recover unreacted phosgene to the upper stage and separate the solvent to the lower stage; and
introducing the recovered unreacted phosgene into the phosgenation reaction step, and sending the solvent separated to the lower stage to the scrubber,
wherein the aliphatic amine is m-xylylene diamine, p-xylylene diamine or o-xylylene diamine, and wherein the phosgenation reaction step comprises a first reaction step of reacting an aliphatic amine and hydrogen chloride to form the aliphatic amine salt; and a second reaction step of reacting the aliphatic amine salt and phosgene in the presence of an organic solvent having a boiling point of 100 to 200°C.

According to the present invention, unreacted materials can be effectively recovered from unreacted materials, solvents and reaction products discharged in the form of a mixture after a phosgenation reaction, and recycled to a reaction step, thereby significantly reducing energy consumed when separating reactants.

### BRIEF DESCRIPTION OF THE DRAWINGS

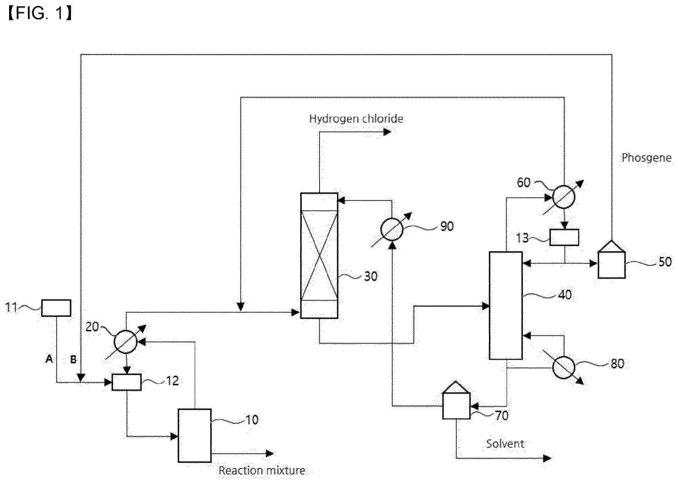

Fig. 1 shows an equipment used in the preparation method of aliphatic isocyanate according to one embodiment of the present invention.

### DETAILED DESCRIPTION OF THE EMBODIMENTS

The terms used herein are only to explain specific embodiments, and are not intended to limit the present invention. A singular expression includes a plural expression thereof, unless it is expressly stated or obvious from the context that such is not intended. As used herein, the terms "comprise", "equipped" or "have", etc. are intended to designate the existence of practiced characteristic, number, step, constructional element or combinations thereof, and they are not intended to preclude the possibility of existence or addition of one or more other characteristics, numbers, steps, constructional elements or combinations thereof.

Although various modifications can be made to the present invention and the present invention may have various forms, specific examples will be illustrated and explained in detail below.

According to the present invention, a method for preparing aliphatic isocyanate is provided, which comprises the steps of: progressing a phosgenation reaction by reacting an aliphatic amine salt and phosgene in the presence of a solvent; separating aliphatic isocyanate, which is a product in the reaction mixture of the phosgenation reaction step, and sending a mixture comprising hydrogen chloride, unreacted phosgene and a solvent to a condenser to condense; sending the condensed mixture to a scrubber to discharge hydrogen chloride to the upper stage and separate unreacted phosgene and solvent to the lower stage; sending the separated unreacted phosgene and solvent to a distillation column to recover unreacted phosgene to the upper stage and separate the solvent to the lower stage; and introducing the recovered unreacted phosgene into the phosgenation reaction step, and sending the solvent separated to the lower stage to the scrubber. The aliphatic amine is m-xylylene diamine, p-xylylene diamine or o-xylylene diamine. The phosgenation reaction step comprises a first reaction step of reacting an aliphatic amine and hydrogen chloride to form the aliphatic amine salt; and a second reaction step of reacting the aliphatic amine salt and phosgene in the presence of an organic solvent having a boiling point of 100 to 200°C.

Hereinafter, the preparation method will be explained according to each step.

First, a phosgenation reaction step is conducted wherein an aliphatic amine salt and phosgene are reacted.

According to the present invention, the phosgenation reaction is conducted by two steps comprising a first reaction step wherein aliphatic amine and hydrogen chloride are reacted first to form an aliphatic amine salt, and a second reaction step wherein the aliphatic amine salt and phosgene are reacted, so as to inhibit side reactions generated during the reaction of aliphatic amine and phosgene and/or generation of side products.

More specifically, the first reaction step is a step wherein an aliphatic amine salt is formed by the neutralization reaction of reacting aliphatic amine and hydrogen chloride, so as to inhibit the rapid reaction with phosgene. The neutralization reaction for the formation of the salt may be conducted at a temperature of 20 to 80°C, for example.

Xylylene diamine exhibits more excellent effect, when applied for the preparation method of aliphatic isocyanate according to the present invention. Specifically, m-xylylene diamine, p-xylylene diamine or o-xylylene diamine is used.

Next, a second reaction step is conducted wherein the aliphatic amine salt and phosgene are reacted.

During the reaction of aliphatic amine salt and phosgene, side reactions are generated, and as side products, for example, monoisocyanate such as chlocomethylbenzylisocyanate(CMBI), and the like are generated. The generation of side reactions and side products are known to be caused due to the necessity for maintaining a high temperature during the preparation process of aliphatic isocyanate, and high reactivity of produced aliphatic isocyanate such as XDI, and the like. Particularly, the final product aliphatic isocyanate, if exposed to a high temperature for a certain time, may cause side reactions or form side products in the form of polymer.

Thus, according to one embodiment of the present invention, when conducting the second reaction step, aliphatic isocyanate may be formed while phosgene is not introduced at once, but is introduced dividedly.

For example, when conducting the second reaction step, at a relatively low temperature, phosgene is primarily introduced in a relatively small amount to produce an intermediate, and while secondarily introducing the remaining amount of phosgene at high temperature, the phosgene and the intermediate are reacted to form aliphatic isocyanate, but the present invention is not limited thereto.

Xylylene diisocyanate(XDI) belonging to aliphatic isocyanate is formed by the reaction of xylylene diamine and phosgene, and first, a small amount of phosgene is primarily introduced and reacted with xylylene diamine salt, thereby producing an intermediate in the form of a carbamoyl-based salt.

Thereafter, while secondarily introducing the remaining amount of phosgene, the phosgene and the intermediate in the form of a carbamoyl-based salt are reacted to form xylylene diisocyanate.

According to the method of one embodiment, a time during which the final product aliphatic isocyanate is exposed to high temperature heat may be minimized, and furthermore, an intermediate may be formed in the reaction step of a relatively low temperature, thereby reducing a time during which a high temperature should be maintained in the whole reaction process. As the result, during the preparation process of aliphatic isocyanate, the generation of side reactions or side products may be significantly reduced.

In addition, since the calorie introduced in the whole process is decreased, the whole process cost may be also reduced. Thus, according to the preparation method of one embodiment, high purity aliphatic isocyanate may be prepared with high yield through a simple preparation process, and furthermore, the high temperature reaction time of phosgene may be relatively reduced to decrease the riskiness of explosive vaporization of phosgene.

Meanwhile, the second reaction stepis progressed in the presence of an organic solvent having a boiling point of 100 to 200°C. When the second reaction step is progressed in the presence of a solvent having a high boiling point, high purity aliphatic isocyanate may be prepare with high yield.

And, the organic solvent may include at least one of an aromatic hydrocarbon-based organic solvent and an ester-based organic solvent.

Specifically, the aromatic hydrocarbon-based organic solvent may be a halogenated aromatic hydrocarbon-based organic solvent such as monochlorobenzene, 1,2-dichlorobenzene, or 1,2,4-trichlorobenzene, and the like.

And, the ester-based organic solvent may be fatty acid ester such as amyl formate, n-butyl acetate, isobutyl acetate, n-amyl acetate, isoamyl acetate, methyl isoamyl acetate, methoxybutyl acetate, sec-hexyl acetate, 2-ethylbutyl acetate, 2-ethylhexyl acetate, cyclohexyl acetate, methylcyclohexyl acetate, benzyl acetate, ethyl propionate, n-butyl propionate, isoamyl propionate, ethyl acetate, butyl stearate, butyl lactate or amyl lactate, and the like; and aromatic carboxylic acid ester such as methyl salicylate, dimethyl phthalate or methyl benzoate, and the like.

More specifically, the organic solvent may include at least one of an aromatic hydrocarbon-based organic solvent and an ester-based organic solvent having a boiling point of 100 to 200°C.

As such, in case the phosgenation reaction is conducted in the presence of an organic solvent, the aliphatic amine salt may be used at a concentration of 20 vol% or less, for example, 1 to 20 vol%, or 5 to 20 vol%. If the concentration of aliphatic amine or a salt thereof exceeds 20 vol%, there is a concern that a large quantity of amine hydrochloride may be precipitated.

In addition, the above explained phosgenation reaction step may be conducted intermittently or continuously, in a reaction equipment comprising a reactor equipped with a rotation axis; a reactant feeding part connected to the inside of the reactor; a heat source for supplying calorie to the reactor; and a product collecting part for collecting the product produced in the reactor.

The preparation method as explained above is suitable for the preparation of xylylene diisocyanate(XDI).

Next, in the reaction mixture of the phosgenation reaction step, aliphatic isocyanate, which is the reaction product of the phosgenation reaction, is separated to the lower part of the reactor, and the remaining mixture comprising hydrogen chloride, unreacted phosgene, and solvents is sent to a condenser connected to the upper part of the reactor and condensed.

In the reaction mixture of the phosgenation reaction step, hydrogen chloride, unreacted phosgene, solvents, and other intermediate side products are included while the gas phase and liquid phase are mixed, besides aliphatic isocyanate to be obtained. Previously, a condenser was not used at the top of a reactor, but inert gas is purged to the reaction mixture to remove unreacted phosgene and hydrogen chloride in the form of gas, and a part of solvents in the reactor, the reaction solution is filtered and desolventized to obtain products, and the remaining solvents are recollected. However, since the above treatment requires continuous supplement of solvents and phosgene, it leads to increase in the production cost. Since an excessive amount of phosgene is used so as to increase the yield of products in the phosgenation reaction, a significant amount of unreacted phosgene is generated, which is discarded as it is, or the equipment for recovery becomes large, and thus, energy use is increased, thus causing increase in the production cost.

Thus, according to the embodiment of the present invention, the remaining mixture except product in the reaction mixture is condensed, hydrogen chloride, unreacted phosgene, and solvents are respectively separated in the condensed mixture, and the separated unreacted phosgene is refluxed to the phosgenation reaction step of the earlier stage and reintroduced, thereby remarkably reducing the amount of fresh phosgene required for a new phosgenation reaction.

And, in the step of condensing the mixture, an optimized condensation temperature range within which total energy consumed in the reflux of the unreacted phosgene and the amount of phosgene reintroduced may be balanced, may be found out to reduce the whole energy consumption.

Fig. 1 briefly shows the equipment used in the preparation method of aliphatic isocyanate according to one embodiment of the present invention.

Referring to Fig. 1, first, a phosgenation reaction step is conducted in a phosgenation reactor 10. Although not shown in detail in Fig. 1, the phosgenation reaction step is conducted by two steps comprising a first reaction step wherein an aliphatic amine and hydrogen chloride are reacted to form an aliphatic amine salt first, and a second reaction step wherein the aliphatic amine salt and phosgene are reacted, as explained above.

Next, in the reaction mixture of the phosgenation reaction step, aliphatic isocyanate, which is the product of the phosgenation reaction, is separately separated to the lower part of the phosgenation reactor 10 together with solvents, and the remaining mixture comprising hydrogen chloride, unreacted phosgene and a part of solvents is sent to a condenser 20 connected to the upper part of the reactor and condensed.

The condenser 20 may be operated such that the temperature of the mixture may become -20°C or more, or -15°C or more, or -10°C or more, and 40°C or less, or 36°C or less, or 0°C or less. If the temperature of the condenser 20 is less than -20°C, freezing may be generated in the condenser, and if the operation temperature exceeds 40°C, solvents and phosgene discharged without condensation may increase. Thus, it is required that the condenser 20 may be operated at a temperature range of -20 to 30°C, preferably -20 to 0°C.

The mixture passing through the condenser 20 is sent to a scrubber 30. To the upper part of the scrubber 30, hydrogen chloride is discharged in the state of gas, and to the lower part, unreacted phosgene and solvents are separated.

The separated unreacted phosgene and solvents are sent to a distillation column 40. To the upper stage of the distillation column 40, unreacted phosgene is recovered, and to the lower stage, solvents are separated. A part of the separated solvents may pass through a solvent recovery vessel 70, cooled by a solvent cooler 90, and sent to the upper stage of the scrubber and used for scrubbing of hydrogen chloride. And, the remaining solvents may be stored in the solvent recovery vessel 70 and used for a reaction producing aliphatic amine salts.

The unreacted phosgene separated in the distillation column 40 is cooled by a distillation column condenser 60, passes through a reflux vessel 13, is stored in a phosgene recovery container 50, and reintroduced in the phosgenation reaction step. The recovery amount of the unrecated phosgene may vary according to the operation temperature of the condenser 20. As the operation temperature of the condenser 20 is lower, recovered unreacted phosgene may increase, but the energy consumption in the condenser 40 may increase. To the contrary, if the operation temperature of the condenser 20 is high, energy consumption may decrease, but unreacted phosgene that can be recovered may decrease. In the present invention, by increasing unreacted phosgene while decreasing energy consumption to secure the amount of refluxed phosgene, the amount of fresh phosgene to be newly introduced in the next batch may be reduced, ultimately reducing total production cost.

The recycled unreacted phosgene may pass through a phosgene storage vessel 12 connected to a phosgene feeding part 11 and be introduced in a phosgenation reactor 10, and the amount A of fresh phosgene may be controlled such that the sum of the amount A of fresh phosgene fed through the phosgene feeding part 11 and the amount B of unreacted phosgene recycled from the phosgene recovery vessel 50 may be constant.

Meanwhile, it may be difficult to completely remove hydrogen chloride by the scrubber 30, and in the unreacted phosgene and solvents separated to the lower stage of the scrubber 30, hydrogen chloride may be still mixed. Thus, in order to separate the remaining hydrogen chloride, a part of the unreacted phosgene separated in the distillation column 40 may be passed through a distillation column condenser 60 and recycled to the scrubber 30.

According to the method for preparing aliphatic isocyanate of the present invention as explained above, input of fresh phosgene may be reduced 60% or more, compared to the conventional process, thus significantly contributing to the reduction of production cost.

Hereinafter, the actions and effects of the invention will be explained in more detail through specific examples of the invention. However, these examples are presented only as the illustrations of the invention, and the scope of the right of the invention is not determined thereby.

### <Example>

### Example 1

Aliphatic isocyanate was prepared using the equipment and process as shown in Fig. 1.

Under room temperature, atmospheric pressure conditions, 500kg of hydrochloric acid and 560kg of xylylenediamine(XDA) were reacted in 6500kg of a solvent of 1,2-dichloribenzene for 4 hours to form 870kg of hydrochloric acid salt of xylylenediamine.

The temperature of the reactor containing 870kg of hydrochloric acid salt of xylylenediamine was raised to 125°C, and while maintaining the temperature, 1359kg of phosgene was introduced into the reactor and stirred for a reaction progress time. The temperature of the condenser 20 at the upper part of the reactor was maintained at - 20°C from the time at which phosgene was introduced until the time reaction finished, and while recycling condensed phosgene and solvents according to the process shown in Fig. 1, phosgene input into the reactor was maintained constant. The reaction solution was stirred until it became transparent. When the reaction solution became transparent, heating was stopped, and the solution was cooled to 80°C, and then, nitrogen bubbling was conducted.

### Example 2

The experiment was conducted by the same method as Example 1, except that the temperature of the condenser 20 at the upper part of the reactor was maintained at - 15°C in the process of preparing aliphatic isocyanate, and 1517kg of phosgene was used for a reaction time.

### Example 3

The experiment was conducted by the same method as Example 1, except that the temperature of the condenser 20 at the upper part of the reactor was maintained at - 10°C in the process of preparing aliphatic isocyanate, and 1673kg of phosgene was used for a reaction time.

### Example 4

The experiment was conducted by the same method as Example 1, except that the temperature of the condenser 20 at the upper part of the reactor was maintained at 36°C in the process of preparing aliphatic isocyanate, and 2993kg of phosgene was used for a reaction time.

### Comparative Example 1

The experiment was conducted by the same method as Example 1, except that the condenser 20 at the upper part of the reactor was not used in the process of preparing aliphatic isocyanate, and 3470kg of phosgene was used for a reaction time.

The phosgene input and energy consumption according to the main process conditions of Examples and Comparative Examples were described in the following Table 1.

**[Table 1]**

| conditions | Example1 | Example 2 | Example 3 | Example 4 | Comparative Example 1 |
|---|---|---|---|---|---|
| Cond.Temp (°C) | -20 | -15 | -10 | 36 | Non |
| CDC.fresh (kg) | 1358.79 | 1517.25 | 1673.08 | 2992.77 | 3470.09 |
| CDC.vent | 30.23% | 37.45% | 43.17% | 68.05% | 70.97% |
| CDC.solu | 10.36% | 9.35% | 8.59% | 4.98% | 4.53% |
| CDC.reacted | 59.41% | 53.20% | 48.25% | 26.97% | 24.50% |
| Recovered Duty (kcal/hr) | 95,563 | 98,987 | 111,187 | 134,575 | 381,020 |

The meanings of the terms used in the Table 1 are as follows:
Cond.Temp: temperature of a condenser 20 at the upper part of a reactor
CDC.fresh: input of fresh phosgene
CDC.vent: amount of unreacted phosgene
CDC.solu: amount of phosgene dissolved in a solvent
CDC.reacted: amount of reacted phosgene
Recovered Duty: calories required for the recovery of phosgene

Referring to Table 1, it can be confirmed that in the case of Examples 1 to 4 wherein a condenser was installed at the upper part of a reactor according to the preparation method of the present invention, the calories required for the recovery of phosgene and the amount of phosgene to be newly introduced were remarkably reduced, compared to Comparative Examples without a condenser.

### <Description of symbols>

- 10:: phosgenation reactor
- 11:: phosgene feeding part
- 12:: phosgene storage vessel
- 13:: reflux vessel
- 20:: condenser
- 30:: scrubber
- 40:: distillation column
- 50:: phosgene recovery vessel
- 60:: distillation column condenser
- 70:: solvent recovery vessel
- 80:: distillation column reboiler
- 90:: solvent cooler

## Claims

1. A method for preparing aliphatic isocyanate comprising the steps of:
progressing a phosgenation reaction by reacting an aliphatic amine salt and phosgene in the presence of a solvent;
separating aliphatic isocyanate, which is a product in the reaction mixture of the phosgenation reaction step, and sending a mixture comprising hydrogen chloride, unreacted phosgene and a solvent to a condenser to condense;
sending the condensed mixture to a scrubber to discharge hydrogen chloride to the upper stage and separate unreacted phosgene and solvent to the lower stage;
sending the separated unreacted phosgene and solvent to a distillation column to recover unreacted phosgene to the upper stage and separate the solvent to the lower stage; and
introducing the recovered unreacted phosgene into the phosgenation reaction step, and sending the solvent separated to the lower stage to the scrubber,
wherein the aliphatic amine is m-xylylene diamine, p-xylylene diamine or o-xylylene diamine, and
wherein the phosgenation reaction step comprises a first reaction step of reacting an aliphatic amine and hydrogen chloride to form the aliphatic amine salt; and a second reaction step of reacting the aliphatic amine salt and phosgene in the presence of an organic solvent having a boiling point of 100 to 200°C.

2. The method for preparing aliphatic isocyanate according to claim 1, wherein the mixture comprising hydrogen chloride, unreacted phosgene and a solvent is condensed to a temperature of -20 to 40°C.

3. The method for preparing aliphatic isocyanate according to claim 2, wherein the mixture comprising hydrogen chloride, unreacted phosgene and a solvent is condensed to a temperature of -15 to 0°C.

4. The method for preparing aliphatic isocyanate according to claim 1, further comprising a step of cooling the recovered unreacted phosgene by a distillation column condenser, before introducing it into the phosgenation reaction step.

5. The method for preparing aliphatic isocyanate according to claim 1, wherein a part of the solvent separated to the lower stage of the distillation column is cooled by a solvent cooler, sent to the upper stage of the scrubber and used for scrubbing of hydrogen chloride, and a part thereof is recycled to a reactor for producing an aliphatic amine salt.

6. The method for preparing aliphatic isocyanate according to claim 1, wherein a part of the unreacted phosgene separated in the distillation column is passed through a phosgene cooler and recycled to the scrubber.

7. The method for preparing aliphatic isocyanate according to claim 1, wherein the concentration of the aliphatic amine salt is 20 vol% or less.

## Patentansprüche

1. Verfahren zur Herstellung von aliphatischem Isocyanat, umfassend die folgenden Schritte:
Durchführen einer Phosgenierungsreaktion durch Reagieren eines aliphatischen Aminsalzes und Phosgen in Gegenwart eines Lösungsmittels;
Abtrennen von aliphatischem Isocyanat, das ein Produkt in der Reaktionsmischung des Phosgenierungsreaktionsschritts ist, und Senden einer Mischung, die Chlorwasserstoff, nicht umgesetztes Phosgen und ein Lösungsmittel umfasst, an einen Kondensator zum Kondensieren;
Senden der kondensierten Mischung an einen Wäscher, um Chlorwasserstoff in die obere Stufe abzulassen und nicht umgesetztes Phosgen und Lösungsmittel in die untere Stufe abzutrennen;
Senden des abgetrennten, nicht umgesetzten Phosgens und Lösungsmittels an eine Destillationskolonne, um nicht umgesetztes Phosgen für die obere Stufe zurückzugewinnen und das Lösungsmittel für die untere Stufe abzutrennen; und
Einführen des zurückgewonnenen, nicht umgesetzten Phosgens in den Phosgenierungsreaktionsschritt und Zuführen des in der unteren Stufe abgetrennten Lösungsmittels zum Wäscher,
wobei das aliphatische Amin m-Xylylendiamin, p-Xylylendiamin oder o-Xylylendiamin ist, und
wobei der Phosgenierungsreaktionsschritt einen ersten Reaktionsschritt, bei dem ein aliphatisches Amin und Chlorwasserstoff zur Bildung des aliphatischen Aminsalzes umgesetzt werden; und einen zweiten Reaktionsschritt, bei dem das aliphatische Aminsalz und Phosgen in Gegenwart eines organischen Lösungsmittels mit einem Siedepunkt von 100 bis 200 °C umgesetzt werden, umfasst.

2. Verfahren zur Herstellung von aliphatischem Isocyanat nach Anspruch 1, wobei das Gemisch enthaltend Chlorwasserstoff, nicht umgesetztes Phosgen und ein Lösungsmittel auf eine Temperatur von -20 bis 40 °C kondensiert wird.

3. Verfahren zur Herstellung von aliphatischem Isocyanat nach Anspruch 2, wobei das Gemisch aus Chlorwasserstoff, nicht umgesetztem Phosgen und einem Lösungsmittel auf eine Temperatur von -15 bis 0 °C kondensiert wird.

4. Verfahren zur Herstellung von aliphatischem Isocyanat nach Anspruch 1, ferner umfassend einen Schritt des Kühlens des zurückgewonnenen, nicht umgesetzten Phosgens durch einen Destillationssäulenkondensator, bevor es in den Phosgenierungsreaktionsschritt eingeführt wird.

5. Verfahren zur Herstellung von aliphatischem Isocyanat nach Anspruch 1, wobei ein Teil des in die untere Stufe der Destillationskolonne abgetrennten Lösungsmittels durch einen Lösungsmittelkühler gekühlt, in die obere Stufe des Wäschers geleitet und zum Waschen von Chlorwasserstoff verwendet wird und ein Teil davon in einen Reaktor zur Herstellung eines aliphatischen Aminsalzes zurückgeführt wird.

6. Verfahren zur Herstellung von aliphatischem Isocyanat nach Anspruch 1, wobei ein Teil des in der Destillationskolonne abgetrennten, nicht umgesetzten Phosgens durch einen Phosgenkühler geleitet und in den Wäscher zurückgeführt wird.

7. Verfahren zur Herstellung eines aliphatischen Isocyanats nach Anspruch 1, wobei die Konzentration des aliphatischen Aminsalzes 20 Vol.-% oder weniger beträgt.

## Revendications

1. Procédé de préparation d'isocyanate aliphatique comprenant les étapes :
de progression d'une réaction de phosgénation en faisant réagir un sel d'amine aliphatique et du phosgène en présence d'un solvant ;
de séparation de l'isocyanate aliphatique, qui est un produit du mélange réactionnel de l'étape de réaction de phosgénation, et d'envoi d'un mélange comprenant du chlorure d'hydrogène, du phosgène n'ayant pas réagi et un solvant à un condenseur pour le condenser ;
d'envoi du mélange condensé vers un épurateur pour évacuer le chlorure d'hydrogène vers l'étage supérieur et séparer le phosgène n'ayant pas réagi et le solvant vers l'étage inférieur ;
d'envoi du phosgène n'ayant pas réagi et du solvant séparés vers une colonne de distillation pour récupérer le phosgène n'ayant pas réagi vers l'étage supérieur et de séparer le solvant vers l'étage inférieur ; et
d'introduction du phosgène n'ayant pas réagi récupéré dans l'étape de réaction de phosgénation, et d'envoi du solvant séparé vers l'étage inférieur vers l'épurateur,
dans lequel l'amine aliphatique est la m-xylylène diamine, la p-xylylène diamine ou la o-xylylène diamine, et
dans lequel l'étape de réaction de phosgénation comprend une première étape de réaction consistant à faire réagir une amine aliphatique et du chlorure d'hydrogène pour former le sel d'amine aliphatique ; et une seconde étape de réaction consistant à faire réagir le sel d'amine aliphatique et le phosgène en présence d'un solvant organique ayant un point d'ébullition de 100 à 200 °C.

2. Procédé de préparation d'isocyanate aliphatique selon la revendication 1, dans lequel le mélange comprenant du chlorure d'hydrogène, du phosgène n'ayant pas réagi et un solvant est condensé à une température de -20 à 40 °C.

3. Procédé de préparation d'isocyanate aliphatique selon la revendication 2, dans lequel le mélange comprenant du chlorure d'hydrogène, du phosgène n'ayant pas réagi et un solvant est condensé à une température de -15 à 0 °C.

4. Procédé de préparation d'isocyanate aliphatique selon la revendication 1, comprenant en outre une étape de refroidissement du phosgène n'ayant pas réagi récupéré par un condenseur à colonne de distillation, avant de l'introduire dans l'étape de réaction de phosgénation.

5. Procédé de préparation d'isocyanate aliphatique selon la revendication 1, dans lequel une partie du solvant séparé à l'étage inférieur de la colonne de distillation est refroidie par un refroidisseur de solvant, envoyée à l'étage supérieur de l'épurateur et utilisée pour l'épuration du chlorure d'hydrogène, et une partie de celui-ci est recyclée vers un réacteur de production d'un sel d'amine aliphatique.

6. Procédé de préparation d'isocyanate aliphatique selon la revendication 1, dans lequel une partie du phosgène n'ayant pas réagi séparée dans la colonne de distillation est passée à travers un refroidisseur de phosgène et recyclée vers l'épurateur.

7. Procédé de préparation d'isocyanate aliphatique selon la revendication 1, dans lequel la concentration du sel d'amine aliphatique est de 20 % en volume ou moins.
